# EUROPEAN PATENT SPECIFICATION

(11) **EP 0 970 922 B1**
(45) Date of publication and mention of the grant of the patent: **05.09.2007**
(21) Application number: 99201896.0
(22) Date of filing: 14.06.1999
(51) Int. Cl.: C02F 11/04, C02F 3/28, C02F 1/44

(54) **Method for methane fermentation of organic waste**
Verfahren für die Methangärung von organischen Abfällen
Procédé de fermentation methanique de déchets organiques

(30) Priority: 06.07.1998 JP 18953598; 15.07.1998 JP 19937098; 18.08.1998 JP 23105698; 18.08.1998 JP 23105798
(43) Date of publication of application: 12.01.2000
(73) Proprietor: Kubota Corporation, Naniwa-ku, Osaka (JP)
(72) Inventor: Moro, Masashi, c/o Kubota Corporation, Osaka (JP); Soeda, Yuji, c/o Kubota Corporation, Osaka (JP); Yamamoto, Tetsuya, c/o Kubota Corporation, Osaka (JP); Shibata, Toshiyuki, c/o Kubota Corporation, Osaka (JP); Madokoro, Taketoshi, c/o Kubota Corporation, Osaka (JP); Ueno, Masaru, c/o Kubota Corporation, Osaka (JP); Komatsu, Toshihiro, c/o Kubota Corporation, Osaka (JP); Wakahara, Shin-ichiro, c/o Kubota Corporation, Osaka (JP)
(74) Representative: Kupecz, Arpad

(56) References cited:
- WO-A-90/15028
- US-A- 5 342 524
- PATENT ABSTRACTS OF JAPAN vol. 1998, no. 02, 30 January 1998 (1998-01-30) & JP 09 271744 A (KAJIMA CORP), 21 October 1997 (1997-10-21)
- BEAUBIEN A ET AL: "Design and operation of anaerobic membrane bioreactors: development of a filtration testing strategy" JOURNAL OF MEMBRANE SCIENCE, ELSEVIER SCIENTIFIC PUBL.COMPANY. AMSTERDAM, NL, vol. 109, no. 2, 24 January 1996 (1996-01-24), pages 173-184, XP004041755 ISSN: 0376-7388
- PATENT ABSTRACTS OF JAPAN vol. 013, no. 362 (C-625), 14 August 1989 (1989-08-14) & JP 01 119397 A (AKUA RUNESANSU GIJUTSU KENKYU KUMIAI), 11 May 1989 (1989-05-11)
- IMASAKA, T ET AL: "APPLICATION OF GAS-LIQUID TWO-PHASE CROSS-FLOW FILTRATION TO PILOT-SCALE METHANE FERMENTATION" DRYING TECHNOLOGY, vol. 11, no. 4, 1993, pages 769-785, XP001032156
- PATENT ABSTRACTS OF JAPAN vol. 014, no. 074 (C-0687), 13 February 1990 (1990-02-13) & JP 01 293183 A (MEIDENSHA CORP), 27 November 1989 (1989-11-27)
- PATENT ABSTRACTS OF JAPAN vol. 015, no. 341 (C-0863), 29 August 1991 (1991-08-29) & JP 03 131397 A (KUBOTA CORP), 4 June 1991 (1991-06-04)
- PATENT ABSTRACTS OF JAPAN vol. 1996, no. 10, 31 October 1996 (1996-10-31) & JP 08 141585 A (KUBOTA CORP), 4 June 1996 (1996-06-04)

## Description

### FIELD OF THE INVENTION

The present invention relates to a technique for processing high-concentration organic waste such as night soil, septic tank sludge, agricultural sludge, sewage sludge, farm animal manure, kitchen refuse and food waste, and more particularly, relates to a method for methane fermentation of organic waste.

### BACKGROUND OF THE INVENTION

Conventionally, anaerobic digestion systems have been available as a method for processing high-concentration organic waste such as night soil, septic tank sludge, sewage sludge and kitchen refuse. The anaerobic digestion systems comprises steps of loading crude material high-concentration organic waste into a fermentation tank, processing the organic waste through fermentation by acid former and methane bacteria, and drawing produced fermented sludge out of the tank.

It is, however, required a long organic-waste retention time in the tank because methane bacteria grows slowly. When fermented sludge is drawn out of the fermentation tank, methane bacteria flow out of the tank together with the fermented sludge. This make it impossible to maintain a high concentration of suspended solids (SS) including the methane bacteria within the methane fermentation tank. To ensure a necessary amount of methane bacteria, it is therefore required to increase the volume of the fermentation tank.

Particularly with high-decomposition ratio materials such as kitchen refuse contained in large amounts, for example, if the SS concentration of the kitchen refuse material is 10% and the kitchen refuse decomposition ratio is 85 - 95%, taking into account the compositions of methane bacteria, then the concentration of suspended solids (SS) including methane bacteria is around 1%.

For this reason, as a method for maintaining a high concentration of methane bacteria in the fermentation tank, there has been available one in which fermented sludge is dehydrated and dehydrated cake is returned to the fermentation tank. However, this method involves a high cost for coagulant polymers used for dehydration and has a possibility of fermentation inhibition due to the polymers, and besides a problem that the dehydrated cake can hardly be solved uniformly.

Another method is that fermented sludge is centrifugally concentrated and returned to the fermentation tank. However, this method is poor in SS recovery rate, large in methane bacteria loss and high in power consumption expense, with a problem that inert matters such as sand and large foreign matters would be recovered preferentially.

In methane fermentation of organic waste, there are some cases where organic matter load may become excessive due to variations of the load amount of organic waste to be introduced into the fermentation tank. In this case, because acid producing bacteria react at a higher rate than methane bacteria, there can be seen accumulation of volatile fatty acids such as acetic acid and propionic acid.

When the concentration of these volatile fatty acids is increased so that the concentration of free volatile fatty acids is increased, the methane fermentation comes to be inhibited, in which case unless the concentration of volatile fatty acids is lowered, the methane bacteria would lose the activeness, where two to three months would be required to recover the methane bacteria to the original state. In such a case, the load of organic waste is reduced or stopped, or otherwise free volatile fatty acids are inhibited from increasing by adding alkali agents.

In the meantime, when the total nitrogen amount (T-N) in the organic waste to be introduced into the fermentation tank is at a high concentration, nitrogen other than that used for composition of bacteria cell bodies result in ammonia nitrogen. If the concentration of this ammonia nitrogen becomes high so that the concentration of free ammonia becomes high, then methane fermentation is inhibited.

An object of the present invention is therefore to provide a method for methane fermentation of organic waste which method uses a membrane for solid-liquid separation of fermented sludge so that the concentration of methane bacteria within the methane fermentation tank is maintained at a high concentration to increase fermentation efficiency and, at the same time, that fermentation inhibition due to volatile fatty acids or ammonia nitrogen can be reduced.

Patent Abstracts of Japan vol. 1998, no. 02, 30 January 1998 & JP 09 271744 A (Kajima Corp), 21 October 1997, discloses a process for the anaerobic treatment of kitchen refuse in a closed fermentation tank.

Beaubien A et al., Journal of Membrane Science, Elsevier Scientific Publ. Company, Amsterdam, NL., vol. 109, no. 2, 24 January 1996 (pages 173-184) relates to design and operation of anaerobic membrane bioreactors: development of a filtration testing strategy.

### DISCLOSURE OF THE INVENTION

The present invention provides a method for methane fermentation of organic waste by introducing high-concentration organic waste to a fermentation tank so that the organic waste is decomposed by methane fermentation, characterized by comprising:
providing a circulatory system made up of the fermentation tank, a membrane separation tank which defines a closed space to insufflate gas generated in the fermentation tank and which has a membrane separation unit submerged inside, and a sludge supply path and a sludge return path for making the two tanks communicated with each other;
putting fermented sludge generated in the fermentation tank into a circulation in the circulatory system;
filtrating the fermented sludge by the membrane separation unit out of the system;
removing permeate filtrated in the membrane separation unit in the membrane separation tank;
drawing out excess fermented sludge from a tank bottom of the fermentation tank;
independently controlling hydraulic residence time of liquid contents of the fermented sludge being remained in the system and sludge residence time of solid contents of the fermented sludge being remained in the system;
supplying dilution water to the fermentation tank;
increasing an amount of the permeate in the membrane separate unit in correspondence to an amount of the dilution water;
removing out of the system fermentation inhibiting substances together with the permeate; thereby
suppressing concentrations of volatile fatty acids and ammonia nitrogen which are the fermentation inhibiting substances down below a specified value; and
holding concentration of suspended solids including methane bacteria in the fermentation tank at a specified value necessary for maintenance of the methane fermentation.

With this constitution, since only the liquid content of the fermented sludge that stays in the system comprising the fermentation tank and the membrane separation tank is taken out as permeate, the hydraulic retention time (HRT) for which the liquid content in the fermented sludge stays in the system, and the sludge retention time (SRT) for which the solid content in the fermented sludge stays in the system can be controlled independently of each other. As a result of this, the concentration of methane bacteria can be maintained at a specified value necessary for the maintenance of methane fermentation and moreover that methane fermentation can be ensured.

In an embodiment of the present invention, the method for methane fermentation of organic waste further comprises holding the concentration of suspended solids (SS) including methane bacteria in the fermentation tank at 1 - 10%, preferably 2.5 - 5.5%, while holding viscosity of the fermented sludge at 200 (mPa · s) or less.

According to the present invention, the method for methane fermentation of organic waste comprises: supplying dilution water to the fermentation tank so that concentrations of volatile fatty acids and ammonia nitrogen as fermentation inhibiting substances are suppressed to below a specified value; and increasing amount of permeate in the membrane separation unit in correspondence to amount of the dilution water so that the fermentation inhibiting substances are removed out of the system along with the permeate.

With this constitution, it becomes possible to reduce the fermentation inhibiting substances in the fermented sludge and maintain the sludge concentration at a high concentration at the same time, so that methane fermentation can be accelerated. The dilution water may be obtained by treating the discharged permeate by means of biological denitrification process, ammonia stripping process, ammonia absorbents or the like, or may be given by process water generated in some other treatment process or pure water.

The concentration of the fermentation inhibiting substances is desirably 3000 mg/L or lower in concentration of ammonia nitrogen.

In an embodiment of the present invention, the method for methane fermentation of organic waste further comprises methane-fermenting organic waste composed of a mixture of kitchen refuse and activated sludge in the fermentation tank.

For example, activated sludge such as excess sludge generated in the biological denitrification process or sewage sludge are microorganisms and therefore, upon the loading of activated sludge to the methane fermentation tank, part of the microorganisms are decomposed under methane fermentation. This decomposition causes trace metals and vitamins to be eluted from the microorganisms into the fermented sludge, and these trace metals and vitamins contribute to stabilization of methane fermentation.

Because activated sludge is slow in decomposition rate, acid fermentation does not progress abruptly upon loading of the activated sludge into the fermentation tank so that volatile fatty acid concentration does not increase abruptly. Therefore, inhibition of methane fermentation due to the volatile fatty acids is unlikely to occur, which leads to stabilization of methane fermentation.

According to the present invention, a complexing agent composed of organic acid is loaded to the methane fermentation tank.

Whereas sufficient trace metals such as Co, Ni, Fe necessary for methane fermentation are normally present in the fermentation tank, most of those trace metals are settled as sulfides in anaerobic atmosphere. The complexing agents loaded into the methane fermentation tank, for example, citric acid or oxalic acid, react with sulfides of the trace metals, which have been settled in the tank, to make the trace metals dissolved and changed into complex ions, thus transforming the trace metals into such a form that microorganisms can make use of the trace metals. As a result of this, methane fermentation is facilitated.

In an embodiment of the present invention, the method for methane fermentation of organic waste further comprises: blowing internal gas generated in the tank into the membrane separation tank from a diffuser disposed below the membrane separation unit, thereby causing a cross-flow along filtration membrane of the membrane separation unit as a result of the gas blow into the tank, so that the fermented sludge is filtrated by the membrane separation unit while cleaning out membrane surfaces of the filtration membrane by the cross-flow.

With this constitution, by cleaning out the membrane surfaces of the filtration membrane of the membrane separation unit with the upward stream, deposition of a cake layer onto the membrane surfaces can be suppressed so that fouling of the filtration membranes can be prevented.

In an embodiment of the present invention, the method for methane fermentation of organic waste further comprises: holding internal pressure of the membrane separation tank at a positive pressure above atmospheric pressure.

With this constitution, internal pressure of the tank is increased to a positive pressure by the gas blown in from the fermentation tank, thus blocking the entrance of external air so that an environment free from the presence of oxygen suitable for the growth of methane bacteria, which are anaerobic bacteria, is maintained. Also, the membrane separation unit submerged in the fermented sludge of the membrane separation tank filtrates the fermented sludge in the tank while a transmembrane pressure that acts on the filtration membrane is utilized as a driving pressure. By holding the internal pressure of the tank to a positive pressure above the atmospheric pressure, the gas pressure in the tank in addition to the water head in the tank acts as a driving pressure that enhances the transmembrane pressure.

### BRIEF DESCRIPTION OF THE DRAWINGS

Fig. 1 is a schematic view showing an arrangement of a methane fermentation tank in an embodiment of the present invention;
Fig. 2 is a perspective view showing an arrangement of a membrane separation unit in the embodiment;
Fig. 3 is a schematic view showing an arrangement of a methane fermentation tank in another embodiment of the present invention;
Fig. 4 is a flow sheet showing the procedure for processing organic waste, showing an embodiment of the present invention;
Fig. 5 is a flow sheet showing the procedure for processing organic waste, showing another embodiment of the present invention;
Fig. 6 is a correlation diagram of sludge concentration and viscosity;
Fig. 7 is a correlation diagram of sludge concentration and viscosity;
Fig. 8 is a graph showing the relation between HRT and methane gas yield;
Fig. 9 is a graph showing the relation between SRT and methane gas yield;
Fig. 10 is a graph showing the relation between ammonia nitrogen concentration and methane gas yield;
Fig. 11 is a graph showing a comparison of methane gas yield between excess sludge and kitchen refuse; and
Fig. 12 is a graph showing the effects of citric acid.

### DETAILED DESCRIPTION OF THE PREFERRED EMBODIMENTS

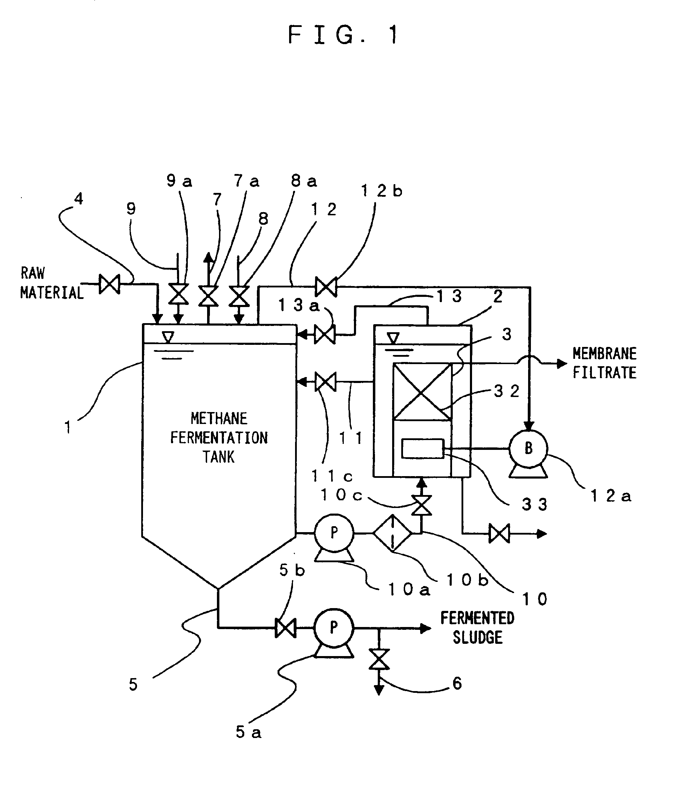

Hereinbelow, an embodiment of the present invention is described with reference to the accompanying drawings. Referring to Fig. 1, a methane fermentation tank 1 and a membrane separation tank 2 define a hermetically closed space, and a membrane separation unit 3 is disposed so as to be submerged in the membrane separation tank 2. The methane fermentation tank 1 and the membrane separation tank 2 may be provided separate from each other or formed by partitioning one tank, or otherwise the membrane separation unit 3 may be submerged directly in the methane fermentation tank 1.

In the methane fermentation tank 1, its suitable temperature environment depends on the type of bacteria, and preferably 30 to 38 °C for mesophilic bacteria and 50 to 57 °C for thermophilic bacteria. In this embodiment, the temperature is maintained at 50 to 57 °C . In this temperature region, kinematic viscosity of the liquid that permeates the membrane is as low as about 1/1.5, as compared with the case of 30 to 38 °C, so that the water head that gives a driving pressure (transmembrane pressure) for performing membrane separation can be reduced. For example, if the temperature in the tank is 55°C and the flux is 0.1 m³/(m² · d), then the transmembrane pressure is 1 to 10 kPa. If the temperature in the tank is 37°C, the transmembrane pressure is 5 to 30 kPa. For maintenance of this temperature, heating equipment (not shown) is provided as an attachment.

In the membrane separation tank 2, although an opening for inspecting the membrane separation unit 3 is closed by a lid member (not shown), it is difficult to completely hermetically close the opening. For growth of methane bacteria in an environment without the presence of oxygen, it is desirable to maintain the interior of the membrane separation tank 2 and thereby block the entrance of external air. In the membrane separation unit 3, a plurality of membrane modules 32 are accommodated vertical and parallel with appropriate spacings in a casing 31, and a diffuser 33 is placed below the membrane modules 32. The method for activating a driving pressure on the membrane separation unit 3 is, desirably, a gravity filtration method making use of the water head, but a suction filtration method for compulsorily activating a suction force is also possible. This membrane separation unit 3 will be described in more detail.

To the methane fermentation tank 1, are connected a material supply line 4 for introducing high-concentration organic waste which is a crude material, and a sludge removal line 5 for taking out fermented sludge. The sludge removal line 5 has a sludge pump 5a and a valve 5b, and a sample takeout hole for taking out a sample of sludge concentration measurement is provided in the downstream side of the sludge pump 5a.

To the top of the methane fermentation tank 1, a gas discharge line 7 for taking out gas including methane gas and contained in the tank is connected via a valve 7a, a dilution water supply line 8 for supplying dilution water is connected via a valve 8a, and an agent supply line 9 for loading a chemical agent of citric acid or oxalic acid is connected via a valve 9a.

Between the methane fermentation tank 1 and the membrane separation tank 2, are provided a sludge supply path 10 for supplying the fermented sludge in the methane fermentation tank 1 to the membrane separation tank 2, and a sludge return path 11 for returning the fermented sludge in the membrane separation tank 2 to the methane fermentation tank 1, where the methane fermentation tank 1, the membrane separation tank 2, the sludge supply path 10 and the sludge return path 11 constitute a circulatory system. The sludge supply path 10 has a circulating pump 10a, a screen 10b and a valve 10c, while the sludge return path 11 has a valve 11c. Also, between the methane fermentation tank 1 and the membrane separation tank 2, are provided a gas supply line 12 for supplying methane gas produced in the methane fermentation tank 1 to the diffuser 33, and a gas return line 13 for returning the methane gas in the membrane separation tank 2 to the methane fermentation tank 1, where the gas supply line 12 has a blower 12a and a valve 12b and the gas return line 13 has a valve 13a.

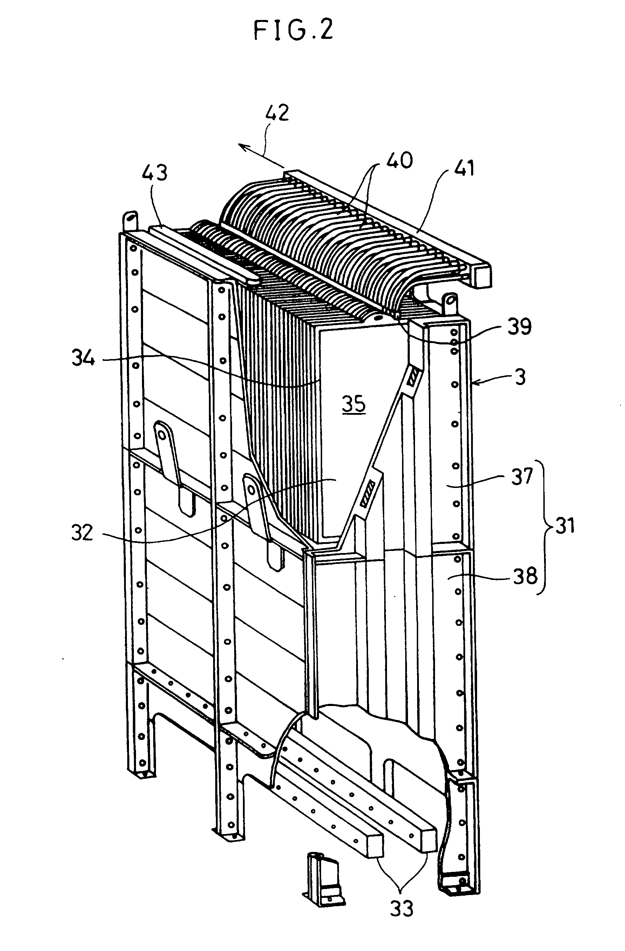

Fig. 2 shows an example of the membrane separation unit 3. In the membrane separation unit 3, a plurality of membrane modules 32 are accommodated vertical and parallel with appropriate spacings inside the casing 31. Each membrane module 32 is made up by placing a filtration membrane 35 over both front and rear surfaces of a filtration plate 34, where the filtration plate 34 is a plate-type rigid member made of ABS resin. The filtration membrane 35 is an ultra-filtration membrane or a microfiltration membrane, preferably being a sheet-like organic membrane whose material is polyethylene and its mean pore size is 0.1 to 0.5 µm in diameter (because the length of methane producing bacteria is not less than 0.5 µm. The membrane module 32 may be a ceramic tubular member or a tube.

The casing 31, in which a plurality of membrane modules 32 and the diffuser 33 are accommodated, is divided into a membrane casing 37 and a diffuser casing 38 for easier transport and maintenance and has such an arrangement that a total amount of gas spouted out from the diffuser 33 enters into the membrane casing 37.

The filtration plate 34 has, in its interior, a permeate path for the flow of permeate liquid that has permeated through the filtration membrane 35. This permeate path has an inflow opening opened at front and rear surfaces of the filtration plate 34 and is communicated with a permeate takeout hole 39 formed at an upper edge of the filtration plate 34. Each membrane module 32 is communicated with a catchment pipe 41 via tubes 40 connected to the permeate takeout hole 39, and a permeate introducing pipe 42 for introducing a permeate liquid is provided in communication with the catchment pipe 41. Reference numeral 43 denotes a holder plate for preventing the membrane modules 32 from floating.

Now, operation of the above-described system is described. High-concentration organic waste loaded into the methane fermentation tank 1 from the material supply line 4 is methane fermented in the tank, and the resulting fermented sludge is circulated to and from the membrane separation tank 2 through the sludge supply path 10 and the sludge return path 11. This circulation of fermented sludge is performed through the screen 10b by opening the valve 10c and a valve 11a and driving the circulating pump 10a. Methane gas in the methane fermentation tank 1 is supplied to the diffuser 33 through the gas supply line 12, spouted out from the diffuser 33 into the fermented sludge of the membrane separation tank 2, and circulated from the membrane separation tank 2 to the methane fermentation tank 1 through the gas return line 13.

With this constitution, pressure in the membrane separation tank 2 is increased to a positive pressure by the methane gas spouted out from the methane fermentation tank 1, thus being blocked from entrance of external air even if the tank is incomplete in hermetic state. Therefore, interior of the membrane separation tank 2 is stably maintained at an environment free from the presence of oxygen suitable for the growth of methane bacteria.

The water head in the membrane separation tank 2 acts as a driving pressure on each membrane module 32 of the membrane separation unit 3, and the membrane separation unit 3 filtrates fermented sludge by the membrane modules 32. In this process, by the tank pressure being maintained at a positive pressure above atmospheric pressure, gas pressure in the tank in addition to the water head in the tank acts as a driving pressure that enhances the transmembrane pressure.

The permeate that has permeated through the filtration membrane 35 of the membrane modules 32 flows from the permeate takeout hole 39 to the tubes 40 through the permeate path of the filtration plate 34, and flows out of the tank through the catchment pipe 41 and the permeate introducing pipe 42.

In this way, by taking out of the system the permeate that has been filtrated by the membrane separation unit 3, the suspended solids (SS) including the methane bacteria in the methane fermentation tank 1 are maintained at a specified concentration value, by which the high-concentration organic waste is methane fermented stably in the methane fermentation tank 1. Fermented sludge, which increases in concentration of methane bacteria with increasing SS concentration, whereas the fermented sludge would deteriorate in fluidity with the viscosity over 200 (mPa · s), making the fermented sludge hard to stir and convey by pump. Therefore, the specified value of SS concentration of fermented sludge is generally 1 to 10%, and preferably 2.5 to 5.5%.

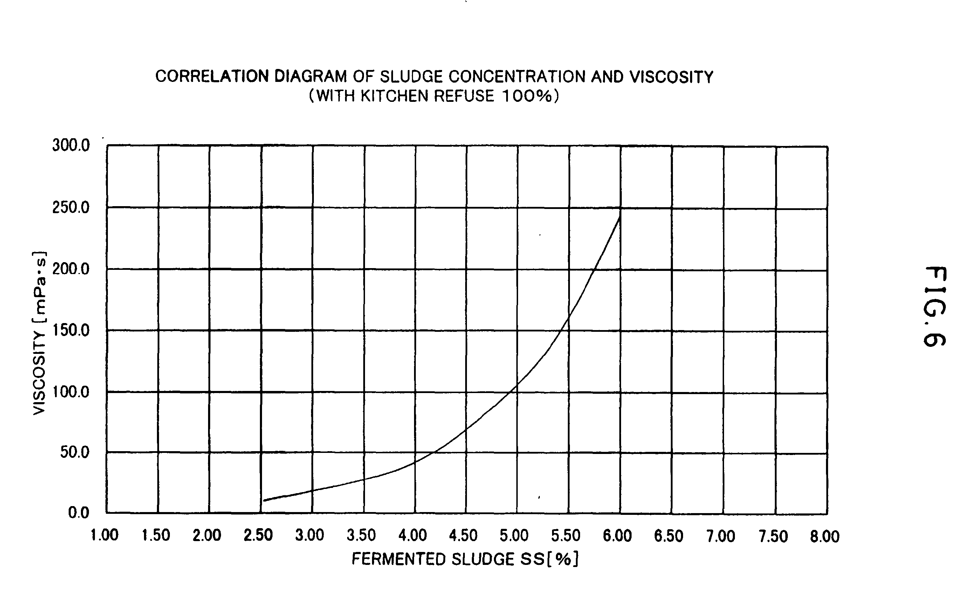

For example, when kitchen refuse having properties as shown in Table 1 is used at 100% as the organic waste, the correlation between SS concentration and viscosity of fermented sludge is as shown in Fig. 6, where in a viscosity range of not higher than 200 (mPa · s), the SS concentration is not more than 5.75%:

**Table 1**

| | | | | |
|---|---|---|---|---|
| Item | Unit | Minimum | Maximum | Average |
| TS | mg/L | 119000 - | 278000 | 191000 |
| VS | mg/L | 108000 - | 252000 | 172000 |
| SS | mg/L | 98000 - | 190000 | 150000 |
| CODcr | mg/L | 102000 - | 371000 | 222000 |
| BOD | mg/L | 69000 - | 260000 | 152000 |
| T-N | mg/L | 1500 - | 21000 | 9600 |
| T-P | mg/L | 350 - | 2000 | 1000 |

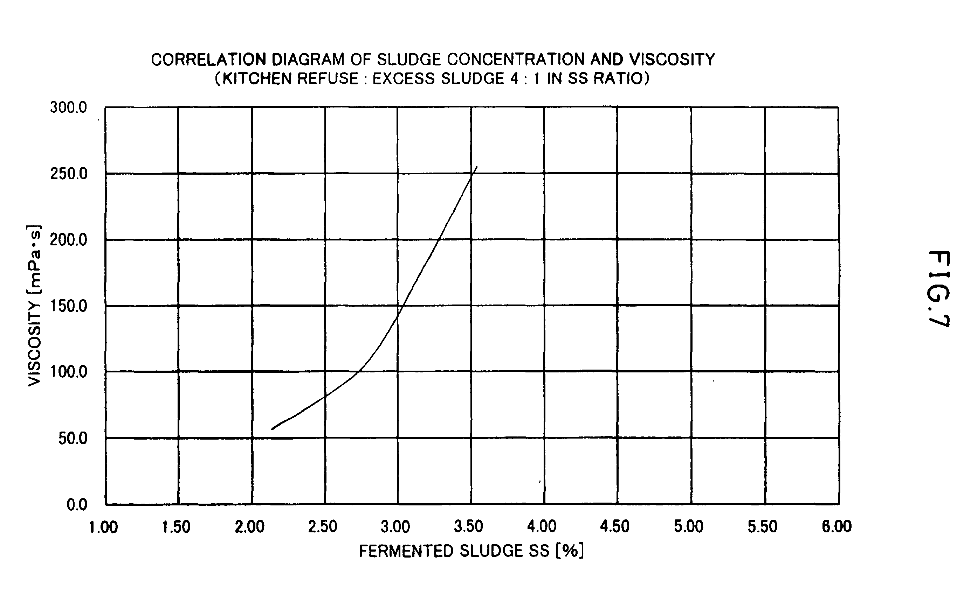

Also, when a mixture of the kitchen refuse shown in Table 1 and excess sludge of night soil treatment shown in Table 2 at an SS quantity ratio of 4 : 1 is used as the organic waste, the correlation between SS concentration and viscosity is as shown in Fig. 7, where in the viscosity range of not higher than 200 (mPa · s), the SS concentration is not more than 3.25%:

**Table 2**

| Item | Unit | Minimum | Maximum | Average |
|---|---|---|---|---|
| TS | mg/L | 17000 - | 30000 | 22500 |
| VS | mg/L | 12000 - | 22000 | 16000 |
| SS | mg/L | 13000 - | 25000 | 18000 |
| CODcr | mg/L | 15000 - | 31000 | 22000 |
| BOD | Mg/L | 900 - | 3500 | 1500 |
| T-N | Mg/L | 1000 - | 2000 | 1300 |
| T-P | Mg/L | 400 - | 700 | 530 |

In the membrane separation tank 2, the total amount of biogas spouted out from the diffuser 33 enters into the diffuser casing 38, and an upward stream in a gas-liquid mixed phase generated by the spout of biogas flows into the membrane casing 37. This upward stream flows as a cross-flow along the filtration membrane 35 of the membrane module 32 to clean out the membrane surface of the filtration membrane 35, thereby suppressing the deposition of a cake layer onto the membrane surface. As a result of this, fouling of the filtration membranes is prevented and reduction of the solid-liquid separation function is suppressed, by which the membrane separation unit 3 is prevented from resulting in malfunction.

Excess fermented sludge in the methane fermentation tank 1 is removed from the tank bottom through the sludge removal line 5. This removal of excess sludge is performed by opening the valve 5b and driving the sludge pump 5a.

In this way, only the liquid content of fermented sludge that stays in the system comprising the methane fermentation tank 1 and the membrane separation unit 3 is taken out as a permeate. Therefore, the hydraulic retention time (HRT) for which the fermented sludge stays in the system, and the sludge retention time (SRT) for which the solid content in the fermented sludge stays in the system can be controlled independently of each other.

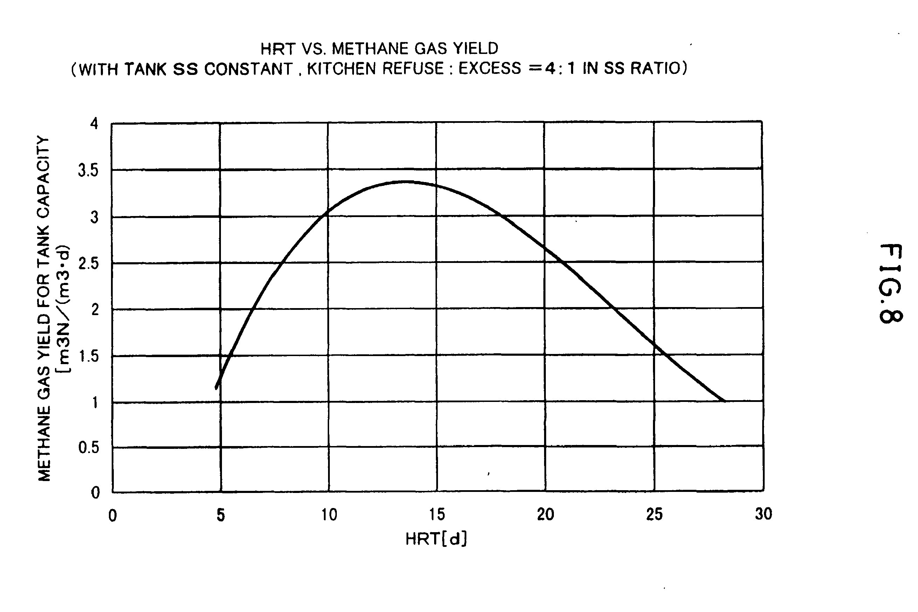

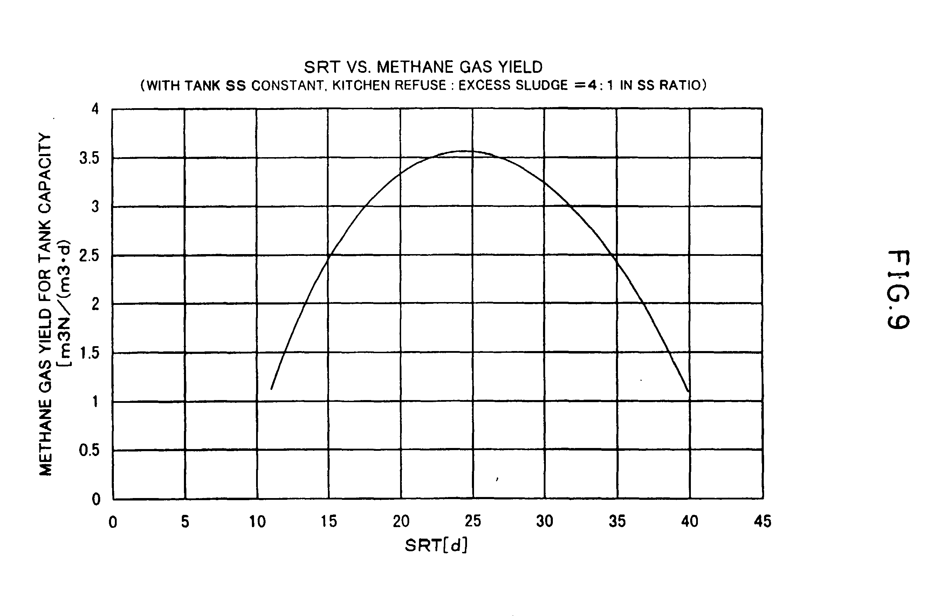

Fig. 8 shows the relation between HRT and methane gas yield in the case where the kitchen refuse shown in Table 1 and the excess sludge of night soil treatment (biological denitrification treatment) shown in Table 2 are mixed at a quantity ratio of 4 : 1, and where the SS concentration in the tank is maintained at a constant value. Fig. 9 shows the relation between SRT and methane gas yield under the same conditions. As shown in Figs. 8 and 9, the methane gas yield shows an HRT peak at 12 to 14 days and an SRT peak at 22 to 26 days.

As shown above, because HRT and SRT differ in optimal value from each other, outflow of methane bacteria can be suppressed by controlling HRT and SRT independently of each other so that the concentration of methane bacteria can be stably maintained at a specified value necessary for maintenance of methane fermentation and moreover that methane fermentation can be stably achieved.

In the methane fermentation tank 1, volatile fatty acids and ammonia nitrogen that are fermentation inhibiting substances are produced by acid producing bacteria. Therefore, with dilution water supplied from the dilution water supply line 8 to the methane fermentation tank 1, the concentration of fermentation inhibiting substances in the fermented sludge is suppressed to below a specified concentration suitable for methane fermentation, and the fermentation inhibiting substances are taken out of the system along with the permeate separated by the membrane separation unit 3. Therefore, the concentration of fermentation inhibiting substances can be reduced to below the specified value without lowering the concentration of methane bacteria in the methane fermentation tank 1.

In this connection, concentrations of free volatile fatty acids and free ammonia at which methane fermentation is inhibited differ depending on such conditions as pH and temperature in the methane fermentation tank 1, breeding state of methane bacteria and the like.

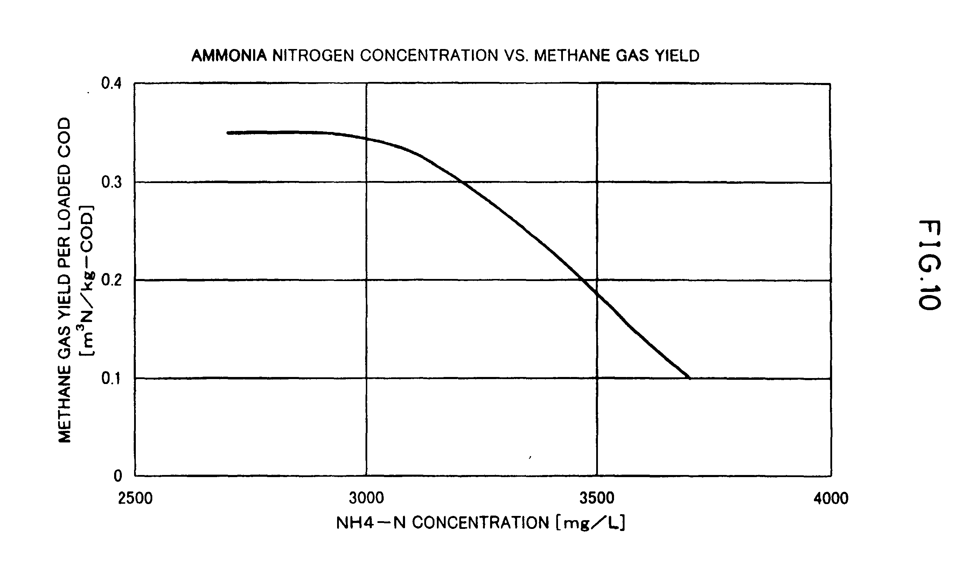

Therefore, a relation between the variation of methane gas yield and the concentration of free volatile fatty acids and free ammonia is determined as a rule of thumb, and an allowable specified concentration of free volatile fatty acids and free ammonia is determined. Fig. 10 shows the relation between ammonia nitrogen concentration and methane gas yield in this embodiment. The concentration of ammonia nitrogen is desirably not more than 3000 mg/L.

Excess sludge produced in night soil treatment is activated sludge composed of microorganisms. When kitchen refuse and the excess sludge are mixed together as organic waste, part of the microorganisms are decomposed together with the kitchen refuse during methane fermentation.

This decomposition causes trace metals and vitamins to be eluted from the microorganisms into the fermented sludge, and these trace metals and vitamins contribute to the cell body composition of methane bacteria, which leads to stabilization of methane fermentation. Amounts of trace metals in the excess sludge are shown in Table 3.

**Table 3**

| Amounts of trace metals in excess sludge | | |
|---|---|---|
| Components | Total (mg/L) | Sol (mg/L) |
| Mg | 470 | 17.4 |
| Fe | 890 | 2.4 |
| Co | 0.3 | ND |
| Ni | 3.1 | 0.1 |
| Mn | 27 | ND |
| Zn | 70.9 | 0.2 |
| Cu | 17 | ND |
| Mo | 0.7 | 0.2 |
| Ca | 3330 | 130 |
| K | 1420 | 1090 |
| Na | 1480 | 1440 |

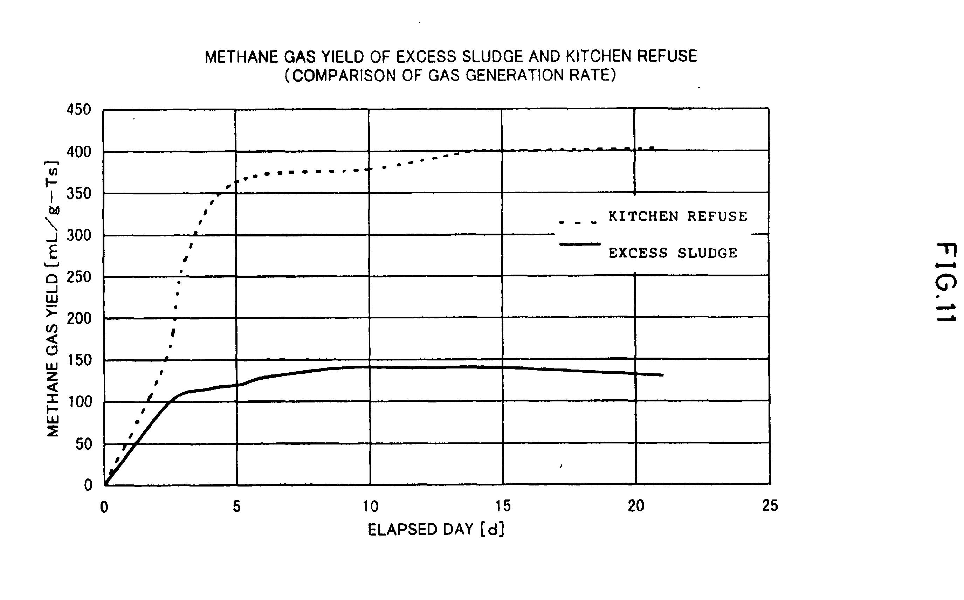

Fig. 11 shows methane gas yields of excess sludge and kitchen refuse. Because excess sludge is slow in decomposition rate, acid fermentation does not progress abruptly upon loading of the excess sludge into the fermentation tank so that volatile fatty acid concentration does not increase abruptly. Therefore, because the concentration of volatile fatty acids does not increase abruptly, inhibition of methane fermentation due to the volatile fatty acids is unlikely to occur, which leads to stabilization of methane fermentation.

Whereas the growth of microorganisms needs a more than specified level of soluble trace metals (Co, Ni, Fe), sufficient trace metals necessary for methane fermentation are present in the fermentation tank as described above. Amounts of trace metals in the fermented sludge are shown in Table 4.

**Table 4**

| Amounts of trace metals in fermented sludge | | |
|---|---|---|
| Components | Total (mg/L) | Sol (mg/L) |
| Mg | 130 | 49.4 |
| Fe | 71.6 | 0.6 |
| Co | 0.2 | 0.1 |
| Ni | 0.4 | 0.1 |
| Mn | 4.7 | 0.3 |
| Zn | 16.2 | 0.2 |
| Cu | 4.8 | 0.1 |
| Mo | 0.4 | 0.1 |
| Ca | 540 | 51.7 |
| K | 740 | 540 |
| Na | 950 | 950 |

However, these trace metals react with carbon dioxide generated by methane fermentation, producing carbonates, or are settled as insoluble sulfides in a reducing atmosphere. In particular, kitchen refuse contains larger amounts of sulfur contents derived from proteins, so that most trace metals are settled as sulfides.

On this account, complexing agents of organic acids such as citric acid or oxalic acid are loaded into the methane fermentation tank 1 through the agent supply line 9. The complexing agents loaded into the methane fermentation tank 1 react with sulfides of the trace metals, which have been settled in the tank, to make the trace metals dissolved and changed into complex ions, thus transforming the trace metals into such a form that microorganisms can make use of the trace metals. As a result of this, methane fermentation is facilitated.

Normally, citric acid and oxalic acid, when used as complexing agents, are loaded by 10 to 100 mg/L, preferably 50 mg/L, relative to the fermented sludge in the tank. Depending on the kind of organic waste or its mixing ratio, the absolute quantity of trace metals may lack, and therefore metal compounds (CoCl₂, NiCl₂, etc.) including Co, Ni, Fe, Mn or the like may be loaded at the same time. Generally, the necessary amount of trace metals is around 1 ppm.

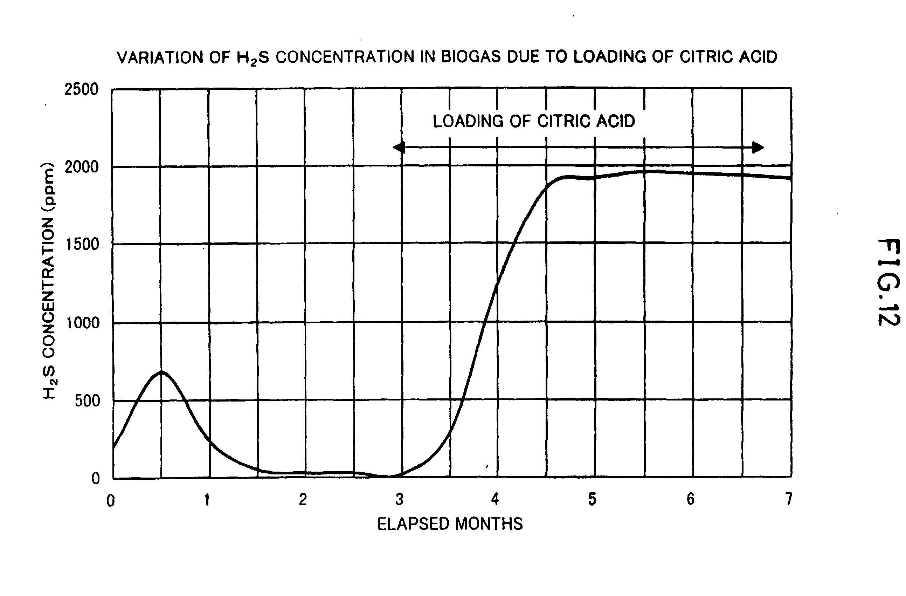

Fig. 12 shows variations of H₂S concentration in biogas attributable to the loading of citric acid, where the increase in H₂S concentration in the biogas shows dissolution and complex-ion formation of sulfides.

In the membrane separation unit 3, sulfides deposit on the filtration membrane 35 of each membrane module 32, causing membrane fouling. On this account, while the membrane separation unit 3 is kept stopped from operation, complexing agents (aqueous solution of 0.1 to 10% concentration) are supplied to the permeate path of the filtration plate 34 through the permeate introducing pipe 42, the catchment pipe 41 and the tubes 40. The state that the complexing agents have penetrated into the filtration membrane 35 is held for an appropriate time elapse (30 to 120 min.), so that the sulfides deposited on the filtration membrane 35 are dissolved. The sulfides are dissolved to form complex ions, thus being changed into such a form as can be utilized by microorganisms, so that methane fermentation is facilitated.

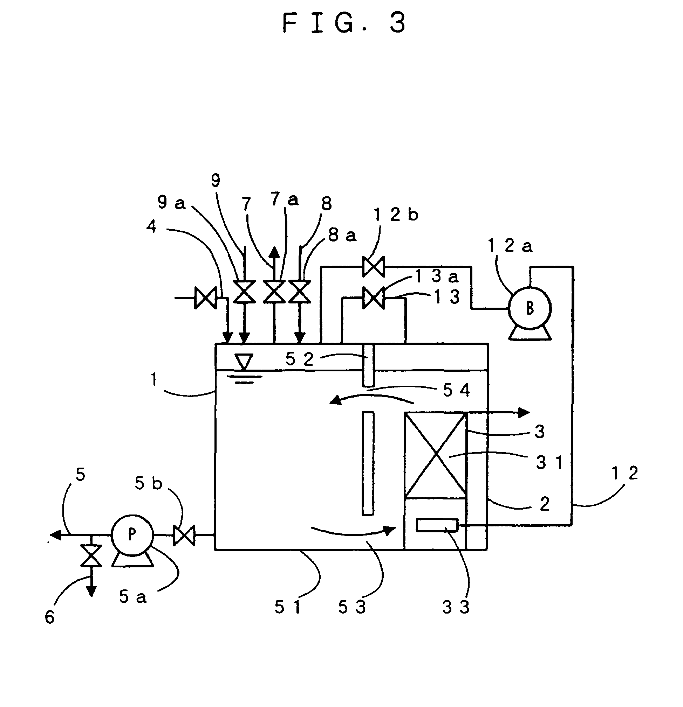

Fig. 3 shows another embodiment of the present invention, where like members functioning as in the foregoing embodiment are designated by like reference numerals and omitted in description. Referring to Fig. 3, a methane fermentation tank 1 and a membrane separation tank 2 are defined by partitioning the interior area of one tank body 51 with a partitioning wall 52, where the methane fermentation tank 1 and the membrane separation tank 2 are communicated with each other at a lower-end opening 53 and an intermediate opening 54 formed in the partitioning wall 52 under the liquid surface.

With this constitution, when methane gas spouts out from a diffuser 33, an upward stream of gas-liquid mixed phase arises and flows into the methane fermentation tank 1 through the intermediate opening 54 of the partitioning wall 52, and fermented sludge of the methane fermentation tank 1 flows into the membrane separation tank 2 through the lower-end opening 53 of the partitioning wall 52. This allows a circulating flow to be formed inside the tank 51 without the need of a separate circulating pump, which in turn allows an agitating action to occur in the methane fermentation tank 1, thus eliminating the need of providing any separate agitating means in the methane fermentation tank 1.

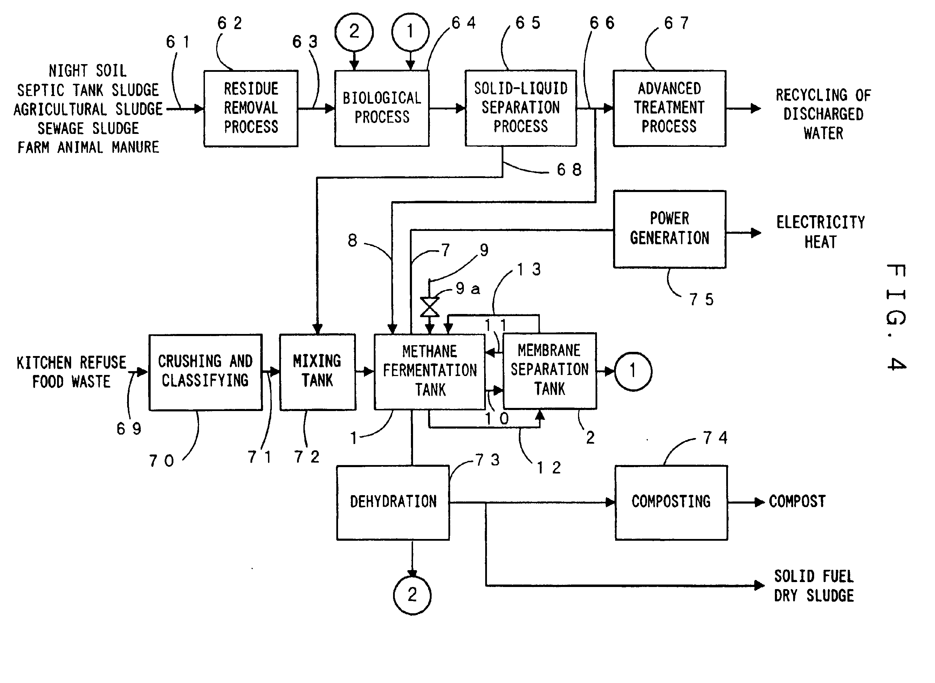

Next, a treatment system in which the above-described methane fermentation tank 1 and membrane separation tank 2 are incorporated is described. Referring to Fig. 4, liquid organic waste 61 such as night soil, septic tank sludge, sewage sludge, agricultural sludge and farm animal manure is introduced to a residue removal process 62 as slurry. In this residue removal process 62, for the protection of a membrane separation unit and a dehydrator in the succeeding stage, it is preferable that after night soil residue is removed by a 0.5 to 5 mm meshed screen as an example, residue removal is performed once again with a centrifugal separator or screw press or the like until even inorganic matters such as fine sands as well as hard-to-solubilize matters such as fibers are separated and removed.

The residue-removed organic waste 63 is subjected to necessary treatment, such as BOD decomposition and denitrification, in a biological treatment process 64. Treatment products processed by the biological treatment process 64 are subjected to separation and removal of suspended substances such as activated sludge in a solid-liquid separation process 65. Treated water 66 after the separation is recycled as dilution water, which will be described later, or subjected to the removal of COD substances, chromaticity components and heavy metals in an advanced treatment process 67 and then disinfected and discharged. Concentrated sludge 68 of the suspended substances such as the activated sludge removed in the solid-liquid separation process 65 is supplied to a mixing tank which will be described later.

Meanwhile, solid organic waste 69 such as kitchen refuse and food waste, which may contain heterogeneous solid contents or include vinyl bags and inorganic matters as fermentation unsuitable matters, is subjected to the removal of inert matters as those unsuitable for fermentation in a crushing and classifying process 70.

The crushing and classifying process 70 is a process in which the organic waste 69 is extruded through a 0.3 to 0.7 mm slit screen with a pressure of 200 to 250 kg/cm² so as to be compressed and crushed with thin-particulate organic waste 71 discharged, while uncrushable, non-biodegradable matters such as night soil residue, plastics, metals and sand are left remaining in the equipment, so that the organic waste 69 is classified into the above two types, and the non-biodegradable matters are dehydrated. It is also possible that coarse crushing may be done as a pretreatment with a crusher such as a uniaxial crusher. The process of classification by compression may be substituted by another process in which crushing is performed with a normal crusher and followed by residue removal with a screen, residue removal with a centrifuge, residue removal with a screw press or the like.

The thin-particulate organic waste (with SS concentration 10 to 15%) 71 discharged from the crushing and classifying process 70, and the aforementioned concentrated sludge (with SS concentration 7 to 10%) 68 are mixed together in a mixing tank 72 and then introduced to the methane fermentation tank 1.

In the methane fermentation tank 1, the fermented sludge is methane fermented while being circulated in conjunction with the membrane separation tank 2. In this process, the solid organic waste 71 has cell membranes destroyed while being formed into finer particles by the crushing under compression, so that the biological decomposition rate of the organic waste 71 is fast, exhibiting an about 20% faster rate as compared with the case in which the crushing under compression is not performed.

Also, the mixture of the thin-particulate organic waste 71 and the concentrated sludge 68 is a mixture in which trace metals (Fe, Ni, Co, Mn, etc.) effective for fermentation contained in kitchen refuse, food waste, septic tank sludge and the like complement one another. Therefore, in the methane fermentation tank 1, the mixture of the thin-particulate organic waste 71 and the concentrated sludge 68 efficiently ferment in smaller numbers of days.

The permeate in the membrane separation unit 3 is supplied to the biological treatment process 64, the excess sludge in the methane fermentation tank 1 is dehydrated in a dehydration process 73, and the supernatant liquor is supplied to the biological treatment process 64. Dewatered sludge in the dehydration process 73 is formed into compost in a composting process 74, or dried and solidified into solid fuel and dry sludge, alternatively, carbonized. Methane gas generated in the methane fermentation tank 1 is introduced to power generating equipment 75 and used as fuel.

When organic matters have become an overload due to variations in the supply amount to the methane fermentation tank 1, volatile fatty acids are abruptly accumulated inside the methane fermentation tank 1 because of the high decomposition rate of acid producing bacteria. Besides, in the methane fermentation tank 1, which is high in total nitrogen concentration (T-N), there is a tendency that ammonia nitrogen is normally accumulated.

On this account, treated water 66 in the solid-liquid separation unit 65 is supplied to the methane fermentation tank 1 as dilution water, so that the concentrations of free volatile fatty acids and free ammonia are suppressed to below specified values, and fermentation inhibiting substances are taken out of the tank together with the permeate separated by the membrane separation unit 3.

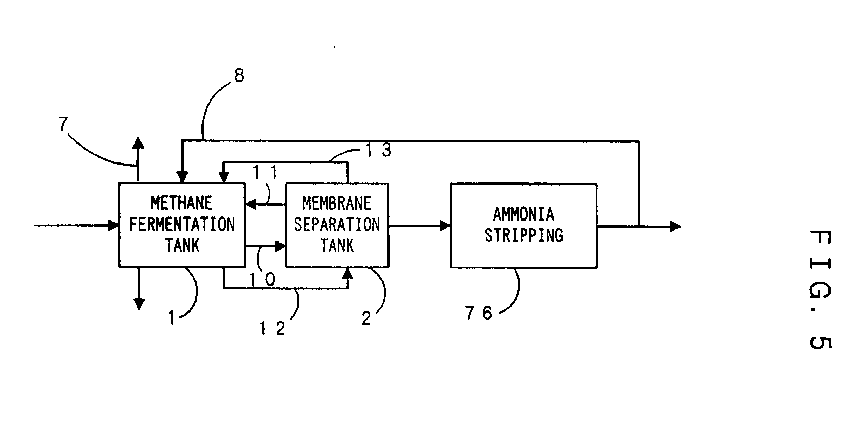

An alternative process is that, as shown in Fig. 5, the permeate taken out from the membrane separation unit 3 is introduced to an ammonia stripping process 76, into which steam is blown in so that ammonia nitrogen in the permeate is radiated as ammonia, where the resulting treated water is used as dilution water. This process has a merit that trace metals or the like necessary for microorganisms are not thinned in concentration, thus giving an advantage in terms of thermal energy.

Without being limited to the above-described embodiments, ammonia contained in the permeate may be removed by zeolite adsorption. As dilution water, process water which is separately prepared may be added. Concentrated sludge may be accelerated for fermentation by performing a solubilizing process in which the concentrated sludge is liquefied and lowered in molecular weight. For the solubilizing process, there are available various methods, exemplified by holding the concentrated sludge for three days at about 70 to 80° C, holding it under high temperature and high pressure of about 70 °C and 0.3 MPa, holding it at about 70 °C with sodium hydroxide, slaked lime or other alkalis, or blowing in ozone gas.

Table 5 shows another embodiment of the present invention, where the SS concentration of kitchen refuse is 20% and the SS concentration of excess sludge is 2.5%.

**Table 5**

| Item | RUN 1 | RUN 2 | RUN 3 | RUN 4 |
|---|---|---|---|---|
| Kitchen refuse (kg/d) | 100 | 100 | 100 | 100 |
| Excess sludge (kg/d) | 0 | 0 | 20 | 20 |
| T-N in material (mg/l) | 3000 | 9000 | 3000 | 9000 |
| Deritrificated water (kg/d) | 0 | 200 | 0 | 180 |
| Fermentation tank capacity (m³) | 1.2 | 1.2 | 1.4 | 1.4 |
| HRT | 12 | 4 | 11.7 | 4.7 |
| SRT | 25 | 25 | 25 | 25 |

RUN 1 and RUN 3 show cases in which the T-N concentration was low, while RUN 3 and RUN 4 show cases in which excess sludge was mixed. In all cases, the SS concentration in the tank was held around 6% and the ammonia nitrogen concentration was held below 3000 mg/L.

The methane gas producing rate on the basis of fermentation tank capacity resulted in around 6 Nm³/m³. This is a very high value for the performance of a fermentation tank that ferments materials having high SS concentration and high total nitrogen concentration. The SS decomposition ratio was able to be held high as much as about 85% for kitchen refuse and about 40% for excess sludge. In this case, no accumulation of volatile fatty acids was seen in the methane fermentation tank.

As shown above, according to the present invention, by subjecting fermented sludge to solid-liquid separation by the membrane separation unit 3, excess water content of the fermented sludge in the methane fermentation tank 1 is removed and the SS concentration in the tank is held above an effective value necessary for the maintenance of methane fermentation, so that stable fermentation can be maintained at all times. As a result, the fermentation tank can be reduced in tank capacity.

By removing inert matters unsuitable for fermentation from the material prior to the loading to the methane fermentation tank 1, active matters occupy a large portion in suspended solids in the methane fermentation tank 1, thus making it possible to improve the fermentation efficiency and to downsize the tank.

By removing impurities through pretreatment of the material, the membrane in the membrane separation unit 3 can be prevented from fouling and damage. Since solid organic waste is formed into fine particles by classification under compression, a high fermentation rate as well as a high SS concentration can be obtained so that the fermentation tank can be downsized by virtue of their multiplier effects.

Fermentation inhibiting substances in the fermented sludge can be easily reduced by filtration, so that methane fermentation can be carried out with high efficiency.

## Claims

1. A method for methane fermentation of organic waste by introducing high-concentration organic waste to a fermentation tank so that the organic waste is decomposed by methane fermentation, **characterized by** comprising:
providing a circulatory system made up of the fermentation tank, a membrane separation tank which defines a closed space to insufflate gas generated in the fermentation tank and which has a membrane separation unit submerged inside, and a sludge supply path and a sludge return path for making the two tanks communicated with each other;
putting fermented sludge generated in the fermentation tank into a circulation in the circulatory system;
filtrating the fermented sludge by the membrane separation unit out of the system;
removing permeate filtrated in the membrane separation unit in the membrane separation tank;
drawing out excess fermented sludge from a tank bottom of the fermentation tank;
independently controlling hydraulic residence time of liquid contents of the fermented sludge being remained in the system and sludge residence time of solid contents of the fermented sludge being remained in the system;
supplying dilution water to the fermentation tank;
increasing an amount of the permeate in the membrane separate unit in correspondence to an amount of the dilution water;
removing out of the system fermentation inhibiting substances together with the permeate; thereby
suppressing concentrations of volatile fatty acids and ammonia nitrogen which are the fermentation inhibiting substances down below a specified value; and
holding concentration of suspended solids including methane bacteria in the fermentation tank at a specified value necessary for maintenance of the methane fermentation.

2. The method for methane fermentation of organic waste according to claim 1, further comprising:
charging into the fermentation tank kitchen refuse and activated sludge which is out of the system;
methane-fermenting the organic waste composed of a mixture of the kitchen refuse and the activated sludge in the fermentation tank; and
charging into the fermentation tank a complexing agent composed of organic acids.

## Patentansprüche

1. Verfahren zur Methangärung von organischem Abfall durch Einbringen von hochkonzentriertem organischen Abfall in einen Gärtank, so dass der organische Abfall durch Methangärung zersetzt wird, **dadurch gekennzeichnet, dass** es umfasst:
Vorsehen eines Zirkulationssystems, das aus dem Gärtank, einem Membrantrenntank, der einen geschlossenen Raum zum Einblasen von im Gärtank erzeugten Gas definiert und der eine in seinem Inneren untergetauchte Membrantrenneinheit aufweist, und einem Schlammzuführweg und einem Schlammrückführweg besteht, welche die zwei Tanks miteinander in Kommunikation bringen;
im Gärtank erzeugter vergorener Schlamm wird im Zirkulationssystem zum Zirkulieren gebracht;
der vergorene Schlamm wird durch die Membrantrenneinheit aus dem System herausgefiltert;
Entfernen von Permeat, das in der im Membrantrenntank befindlichen Membrantrenneinheit herausgefiltert wurde;
Herausziehen von überschüssigem vergorenen Schlamm aus einer Tankunterseite des Gärtanks;
unabhängiges Steuern der hydraulischen Verweilzeit der flüssigen Bestandteile des im System verbliebenen Gärschlamms und der Schlammverweilzeit von festen Bestandteilen des im System verbliebenen Gärschlamms;
Zuführen von Verdünnungswasser zum Gärtank;
Vergrößern einer Menge an Permeat in der Membrantrenneinheit in Übereinstimmung mit einer Menge an Verdünnungswassers;
die Gärung hemmende Substanzen werden zusammen mit dem Permeat aus dem System entfernt; und **dadurch**
werden die Konzentrationen flüchtiger Fettsäuren und Ammoniak-Stickstoff, die gärungshemmende Substanzen sind, bis auf einen festgelegten Wert abgesenkt; und
die Konzentration suspendierter Feststoffen einschließlich Methanbakterien wird im Gärtank auf einem festgelegten Wert gehalten, der zum Aufrechterhalten der Methangärung erforderlich ist.

2. Verfahren zur Methangärung von organischem Abfall nach Anspruch 1, welches weiter umfasst:
in den Gärtank werden Küchenabfälle und aktivierter Schlamm eingebracht, der von außerhalb des Systems ist;
eine Methangärung des organischen Abfalls, der aus einer Mischung aus den Küchenabfällen und dem aktivierten Schlamm besteht, wird im Gärtank durchgeführt; und
in den Gärtank wird ein Komplexbildner eingebracht, der aus organischen Säuren aufgebaut ist.

## Revendications

1. Procédé de fermentation méthanique de déchets organiques, dans lequel on introduit des déchets organiques hautement concentrés dans une cuve de fermentation de manière à ce que les déchets organiques s'y décom-posent par fermentation méthanique, lequel procédé est **caractérisé en ce qu'**il comporte les opérations suivantes :
- prendre un système à circulation, constitué de la cuve de fermentation, d'une cuve de séparation sur membrane, qui définit un espace fermé où est envoyé le gaz produit dans la cuve de fermentation et qui comporte une unité de séparation sur membrane qui s'y trouve submergée, et d'une canalisation d'arrivée de boue et d'une canalisation de retour de boue qui font communiquer les deux cuves entre elles ;
- mettre en circulation, dans ce système à circulation, de la boue fermentée produite dans la cuve de fermentation ;
- filtrer la boue fermentée, au moyen de l'unité de séparation sur membrane, hors du système ;
- enlever le perméat issu de la filtration effectuée dans l'unité de séparation sur membrane, dans la cuve de séparation sur membrane ;
- retirer du fond de la cuve de fermentation l'excès de boue fermentée ;
- réguler indépendamment le temps de séjour des liquides contenus dans la boue fermentée qui reste dans le système et le temps de séjour des solides contenus dans la boue fermentée qui reste dans le système ;
- envoyer de l'eau dans la cuve de fermentation, pour diluer le contenu de celle-ci ;
- augmenter la quantité de perméat obtenue dans l'unité de séparation sur membrane, d'une quantité correspondant à celle de l'eau de dilution ;
- enlever du système, avec le perméat, les substances inhibant la fermentation, ce qui permet de réduire à des valeurs inférieures aux niveaux spécifiés les concentrations d'acides gras volatils et d'azote ammoniacal, qui sont des substances inhibant la fermentation ;
- et maintenir à un niveau spécifié, nécessaire à l'entretien de la fermentation méthanique, la concentration de solides en suspension, y compris les bactéries méthanogènes, dans la cuve de fermentation.

2. Procédé de fermentation méthanique de déchets organiques, conforme à la revendication 1, qui comporte en outre les opérations suivantes :
- charger dans la cuve de fermentation des déchets de cuisine et de la boue activée, qui se trouve hors du système ;
- provoquer, dans la cuve de fermentation, la fermentation méthanique des déchets organiques constitués du mélange des déchets de cuisine et de la boue activée ;
- et charger dans la cube de fermentation un agent complexant composé d'acides organiques.
